# EUROPEAN PATENT APPLICATION

(11) **EP 4 582 112 A1**
(43) Date of publication of application: **09.07.2025**
(21) Application number: 24305004.4
(22) Date of filing: 02.01.2024
(51) Int. Cl.: A61L 31/02, A61C 8/00, A61L 31/08

(54) **MEDICAL DEVICE OR MEDICAL DEVICE FOR COMPONENT AND PROCESS FOR PREPARING IT**

(71) Applicant: ANTHOGYR, 74700 Sallanches (FR); Centre National de la Recherche Scientifique, 75016 Paris (FR); UNIVERSITE CLAUDE BERNARD - LYON 1, 69100 Villeurbanne (FR); Institut National des Sciences Appliquees de Lyon (Insa Lyon), 69100 Villeurbanne (FR)
(72) Inventor: LIENS, Aléthéa, 68128 VILLAGE-NEUF (FR); DOUEST, Yohan, 74380 BONNE (FR); COURTOIS, Nicolas, 74190 PASSY (FR); CHEVALIER, Jérôme, 69140 RILLIEUX-LA-PAPE (FR); FABREGUE, Damien, 42800 CHATEAUNEUF (FR); TER-OVANESSIAN, Benoit, 69100 VILLEURBANNE (FR)
(74) Representative: Ernest Gutmann - Yves Plasseraud S.A.S.

(57) **Abstract**

The present invention relates to a medical device or medical device component comprising a core body made of an amorphous alloy, wherein the main alloy component of the amorphous alloy is selected from the group consisting of titanium copper (TiCu) and zirconium copper (ZrCu). The device of the invention is characterized in that it further comprises a coating formed on at least a part of a surface of the core body, the coating containing a diamond-like carbon (DLC) layer.

## Description

The present invention relates to a medical device or medical device component according to the preamble of claim 1 as well as to a process for preparing the medical device or medical device component.

Medical devices, such as surgical devices or dental implants or even instruments, are typically made of a material, which is biocompatible and exhibits mechanical properties meeting the respective requirements of the device.

Dental implants, for example, are typically made of titanium or of a ceramic material, in particular a zirconia-based ceramic. In addition, dental implants made of a titanium alloy have been successfully introduced to the market owed to the material's excellent combination of high strength, high corrosion resistance and sufficient biocompatibility.

Despite the excellent mechanical properties of titanium alloys currently in use, further downsizing of dental implants as well as other medical devices is limited.

To meet the need in modern surgery for further downsizing medical devices without compromising the mechanical properties to an unacceptable extent, amorphous metals, and in particular Bulk Metallic Glasses, have recently gained increasing attention.

For example, CN110464497A relates to a dental implant made of a titanium-based amorphous metal, which is characterized by including: 30-75 parts of titanium, 0-25 parts of zirconium, 0-45 parts of copper, 0-20 parts of silicon, 0-10 parts of iron, 0-10 parts of zinc, 0-5 parts of silver, and 0-15 parts of palladium.

Similarly, CN110464498A relates to a zirconium-based amorphous metal dental implant, which is characterized by including: 30-65 parts of zirconium, 0-25 parts of titanium, 0-45 parts of copper, 0-20 parts of aluminum, 0-20 parts of silicon, 0-10 parts of iron, 0-10 parts of zinc, and 0-5 parts of palladium.

Amorphous metals of the composition disclosed in CN110464497A have been found to have the potential of exhibiting a compressive yield strength and a fatigue resistance much higher than that of Ti-6Al-4V. In addition, this material has been found to present a high biocompatibility. Thus, the material represents an interesting candidate for applications where a downsizing of an item is aimed at but where a decrease in the mechanical properties must be avoided or at least kept low.

Specifically, Ti₄₀Zr₁₀Cu₃₆Pd₁₄ Bulk Metallic Glass (BMG) has been reported to be attractive for future biomedical applications thanks to its high glass forming ability, the absence of toxic elements and its good mechanical properties (A. Liens et al., "On the Potential of Bulk Metallic Glasses for Dental Implantology: Case Study on Ti40Zr10Cu36Pd14", Materials 2018, 11, 249)). This article also reports on the higher corrosion potential of the BMG compared to Ti-6Al-4V alloy, indicating that more energy is required to initiate the corrosion reaction for the BMG.

However, the article also reports on the Ti-based glassy (amorphous) alloy to suffer from pitting corrosion. This unwanted effect is explained in the article referenced above by the presence of copper oxide in the passive layer surface of the BMG and the finding that copper drastically decreases the corrosion resistance of metallic alloys and metallic glasses.

Although the detrimental effects of the material suffering from pitting corrosion are not fully understood yet, it would in view of the material's use for a medical device be desirable to reduce or even eliminate this effect while maintaining the desirable properties in terms of mechanical stability and biocompatibility.

In view of the drawbacks mentioned above, the object of the present invention is thus to provide a medical device or medical device component, which in terms of mechanical properties and biocompatibility has the favorable properties of an amorphous alloy, in particular a Ti-based BMG such as Ti₄₀Zr₁₀Cu₃₆Pd₁₄, but which simultaneously exhibits a reduced tendency to suffer from pitting corrosion.

This object is solved by the subject matter of claim 1. Preferred embodiments of the present invention are defined in the dependent claims.

According to claim 1, the present invention thus relates to a medical device or medical device component comprising a core body made of an amorphous alloy, wherein the main alloy component of the amorphous alloy is selected from the group consisting of titanium copper (TiCu) and zirconium copper (ZrCu). In particular, the amorphous alloy is in the form of a Bulk Metallic Glass, i.e. an amorphous alloy in bulk form.

The medical device or medical device component of the present invention further comprises a coating formed on at least a part of a surface of the core body, the coating containing a diamond-like carbon (DLC) layer.

In the course of the present invention, it has been found that by the formation of a coating containing a DLC-layer on the surface of the core body, the material's tendency to suffer from pitting corrosion can be reduced substantially. Ultimately, this allows to obtain a medical device which exhibits exceptional mechanical properties and which remains stable even if kept in a corrosive environment.

This in turn makes it possible to further downsize existing medical devices, which despite their reduced dimensions exhibit sufficient mechanical properties and which remain stable over time.

The term "Bulk Metallic Glass" or "BMG" as used in the context of the present invention relates to an amorphous alloy (synonymous to a metallic glass) in bulk form. Specifically, the term "in bulk form" has in this regard the meaning that the amorphous alloy can be produced in a fully dense and amorphous rod having a diameter of at least 1 mm.

The term "diamond-like carbon" or "DLC" as used in the context of the present invention relates to a metastable form of amorphous carbon containing a significant fraction of sp3 bonds (as for example described by J. Robertson, "Diamond-like amorphous carbon, Materials Science and Engineering R 37 (2002) 129-281). More specifically, the "DLC" as used in the context of the present invention relates to an amorphous, partially hydrogenated carbon (in general referred to as a -C:H), in which some of the valence electrons of the carbon atoms are in sp3 configuration and some are in sp2 configuration. For a detailed specification of this DLC film, it is referred VDI-Richtlinien VDI 2840, Kohlenstoffschichten; Grundlagen, Schichttypen und Eigenschaften/Carbon films; Basic knowledge, film types and properties, Verein Deutscher Ingenieure, June 2021, in particular to chapter 5.2.4 (page 18, first bulletpoint) .

For the purpose of the present invention, a titanium-based amorphous alloy or BMG (i.e. an amorphous alloy or BMG containing titanium and copper as main alloy components) is preferred, since it offers an exceptional specific strength, a high corrosion resistance, a high hardness, a low Young's modulus, as well as a ductility and processing capabilities, which are particularly advantageous for medical devices.

Among the titanium-based amorphous alloys or BMGs, a BMG of the Ti-Zr-Cu-Pd system is particularly preferred for biomedical applications, primarily owed to the absence of toxic elements, such as nickel (Ni) or beryllium (Be). In addition, amorphous alloys or BMGs of this system have been found to exhibit a glass forming ability large enough to machine dental implants and dental instruments used in oral implantology without compromising their outstanding mechanical properties.

According to a specific embodiment of the present invention, the amorphous alloy contains titanium and copper as the main alloy components and further contains at least one secondary alloy component selected from the group consisting of zirconium (Zr), palladium (Pd), and mixtures thereof. In this regard, it is further preferred that the amorphous alloy, and in particular the Bulk Metallic Glass, further contains at least one tertiary alloy component selected from the group consisting of iron (Fe), gallium (Ga), tin (Sn), silicon (Si), yttrium (Y), silver (Ag), scandium (Sc), sulfur (S), niobium (Nb), hafnium (Hf), zinc (Zn), tantalum (Ta), and mixtures thereof.

More specifically, the amorphous alloy contains 15-55 weight-% of titanium, 1-45 weight-% of copper, 0-30 weight-% of zirconium, 0-30 weight-% of palladium, 0-10 weight-% of silicon, 0-20 weight-% of iron, 0-10 weight-% of zinc, and 0-20 weight-% of silver, based on the total weight of the amorphous alloy.

In this regard, it is further preferred that the amorphous alloy, and in particular the Bulk Metallic Glass, contains
20-45 weight-%, preferably 25-35 weight-%, of titanium,
15-45 weight-%, preferably 30-40 weight-%, of copper,
5-30 weight-%, preferably 10-20 weight-% of zirconium,
0-30 weight-%, preferably 0-25 weight-% of palladium,
0-15 weight-%, preferably 0-10 weight-% of tin,
0-2 weight-%, preferably 0-1 weight-% of silicon,
0-10 weight-%, preferably 0-5 weight-% of iron, and
0-15 weight-%, preferably 0-10 weight-% of silver,
based on the total weight of the amorphous alloy.

Most preferably, the amorphous alloy, and in particular the Bulk Metallic Glass, is of the composition Ti₄₀Zr₁₀Cu₃₆Pd₁₄, whereby the suffixes relate to the atomic percentages of the respective components. This material has been found to exhibit particularly good mechanical properties, in particular a much higher strength and a much lower Young's modulus compared to a Ti-6Al-4V alloy currently in use.

Surprisingly, the coating according to the present invention has been found to exhibit a high scratch resistance and hence a very good adhesion when formed on an amorphous alloy. In particular, the scratch resistance and the adhesion to the substrate has been found to be improved compared to the coating being formed onto a crystalline alloy, such as Ti-6Al-4V.

In addition, it has been found that the coating shows particularly good adhesion on the surface of the amorphous alloy, if formed directly on the surface of the core body. In other words, no additional silicon or titanium layer mediating bonding between the materials is required.

According to a particularly preferred embodiment, the coating further contains an intermediate layer formed between the DLC layer and the surface of the core body. Said intermediate layer optionally may comprise chromium. The intermediate layer according to this embodiment has been found to function as an adhesive layer, providing particularly good adhesion and, thus, a particularly high scratch resistance of the coating.

Specifically, the intermediate layer is formed directly adjacent to the surface of the core body and the DLC layer, and thus without any further intermediate layers.

Typically, the coating has a thickness in the range of from 1 to 5 um, preferably of from 2 to 4 µm, and most preferably of about 3 um. If an intermediate layer is present, the intermediate layer typically has a thickness in the range from 250 to 750 nm, preferably of about 500 nm, with the DLC layer having a thickness in the range of from 1.25 to 3.75 µm, preferably of about 2.5 um.

In the context of the present invention, the term "thickness" relates to an average thickness of the coating or of the respective layer, taking into account that, typically, the thickness is not perfectly uniform and that the methods for determining the thickness of the coating or layer may for one surface area lead to a value slightly deviating from the one determined for another area. The term "about" as used in the context of the present invention thus relates to a possible deviation from the average of the indicated thickness by up to +/- 5%.

According to a preferred embodiment, the DLC layer contains a silicon-based inner layer region which is an inner DLC layer region containing silicon as a main component. The DLC layer also comprises an outer DLC layer region containing a carbon-based outer layer region which is an outer layer region containing carbon as a main component. More preferably, the proportion of silicon contained in the inner DLC layer region decreases gradually in the direction of the outer DLC layer region and the proportion of carbon contained in the outer DLC layer region decreases gradually in the direction of the inner DLC layer region. Thus, a particularly good adhesion can be established, owed to the silicon-based inner region showing improved adhesion properties compared to the carbon-based outer region.

Preferably, the inner DLC layer region has in this embodiment a thickness in the range of from 250 nm to 750 nm, preferably of from 400 nm to 600 nm, and more preferably of about 500 nm. The outer DLC layer region has in this embodiment preferably a thickness in the range of from 1 µm to 3 µm, and more preferably of about 2 um.

In the context of the present invention, it has further been found that the lowering of the material's susceptibility to pitting corrosion may to a certain degree also be governed by the surface topography of the core body. Preferably, the surface of the core body on which the coating is formed has an arithmetical mean roughness Sa of less than 1 um, preferably less than 0.5 µm, more preferably less than 0.3 um, and even more preferably less than 0.2 µm.

The present invention encompasses any medical devices, and in particular any surgical and dental devices, and components thereof. In consideration of the advantages pointed out above, the medical device or medical device component is preferably a surgical device or a dental implant, prosthesis or abutment, preferably a dental implant supported prosthesis or abutment, more preferably a screw for use in a dental implant supported prosthesis or abutment, or a surgical or a dental instrument, preferably a dental instrument, more preferably a dental driving tool, or a handpiece for use in surgical or dental procedures, in particular a handpiece for dental procedures, or a motor for use in surgical or dental procedures, in particular a motor for use in dental procedures.

The present invention is particularly suitable for dental instruments, since for these instruments a relative smooth core body can be used, as opposed to a dental implant, which in general requires a surface topography of a certain roughness for obtaining a high osseointegration (i.e. a fast, strong and stable interaction of the implant with the surrounding bone tissue). Since the corrosion resistance is particularly pronounced for a medical device comprising a relatively smooth core body, the medical devices or medical device components prepared according to the present invention, in particular the dental instruments obtained, have the advantage to remain corrosion resistant even after a long-term exposure to a humid and warm environment.

According to a particularly preferred embodiment, the medical device is a dental driving tool. Nonetheless, the medical device can also be a dental implant, which can in particular be preferred in cases where a smaller implant size is aimed at, as discussed above.

According to a further aspect, the present invention further relates to a process for preparing the medical device or medical device component as described above, wherein the process comprises the steps of
a) providing a core body made of an amorphous alloy wherein the main alloy component of the amorphous alloy is selected from the group consisting of titanium copper (TiCu) and zirconium copper (ZrCu), and
b) applying on the surface of the core body a coating containing a diamond-like carbon (DLC) layer.

In a preferred embodiment of the invention, the diamond-like carbon (DLC) layer can be applied onto the surface of the core body using a vapor deposition method, in particular using Chemical Vapor Deposition (CVD), Physical Vapor Deposition (PVD), Plasma-Enhanced Vapor Deposition (PECVD) or combinations of these methods.

According to particularly preferred embodiment, the DLC layer is applied onto the surface of the core body by Plasma-Enhanced Vapor Deposition (PECVD; also referred to as Plasma-Activated Chemical Vapor Deposition or "PACVD"), which is a combination of CVD and PVD. The preference for PECVD is owed to the relatively low process temperatures of this method, which can e.g. be lower than 250°C and thus well below the glass temperature of the amorphous alloy, in particular the Ti₄₀Zr₁₀Cu₃₆Pd₁₄, which is a preferred material of the present invention, as discussed above.

Hence, the DLC layer is, according to a further preferred embodiment, applied onto the surface of the core body using a vapor deposition method at a temperature of below 300°C, preferably of below 250°C.

In line with the description of a preferred embodiment of the medical device or medical device component referred to above, it is further preferred that step b) comprises the sub-steps of
b.1. applying on the surface of the core body an intermediate layer, said first layer containing chromium as a main component.

In this regard, it is further preferred that step b) comprises in addition the sub-steps of
b.2. applying on the intermediate layer an inner DLC layer region, said inner DLC layer region containing silicon as a main component, and
b.3. applying on the inner DLC layer region an outer DLC layer region, said outer DLC layer region containing carbon as a main component.

As discussed above, both the inner DLC layer region and the outer DLC layer region are preferably applied by using PECVD.

In particular in the case, in which a machined core body is provided in step a), it is preferred that the method further comprises the step of polishing the core body prior to step b) .

By this polishing, debris and/or burrs clinging onto the surface are efficiently removed. Ultimately, uncovered areas, which may result from debris and/or detaching from the surface after step b), can be efficiently avoided or at least decreased.

As will be discussed in further detail in the context of the working examples below, a specific method of the present invention comprises the steps of
- cleaning the surface of the core body to be treated by ion bombardment, e.g. using nitrogen (N);
- carrying out a PVD step to deposit by ion bombardment an intermediate layer comprising chromium, whereby a bias current is applied on the target;
- carrying out a PECVD step, involving plasma activation of silicone-based precursor gases promoting the precipitation of the inner DLC layer region on the surface, followed by injection of a carbon-based gas (e.g. C₂H₂) in the vacuum chamber to deposit the outer DLC layer region

More specifically, the whole process is conducted at a temperature of below 300°C, preferably of below 250°C.

### EXAMPLES

The disclosure is further illustrated by the following examples, which are not to be construed as limiting this disclosure in scope or spirit, it is to be understood that the examples are provided to illustrate certain embodiments.

Unless otherwise noted, reagents and solvents were used as received from commercial suppliers.

The present invention is further illustrated by way of the following working examples along with the corresponding figures, of which
- Fig. 1: shows the anodic potentiodynamic polarization curves of a body containing a core body made of a Ti-based BMG and a coating according to the present invention in a chlorine containing environment compared to a body solely consisting of the Ti-based BMG core body (without a coating);
- Fig. 2: shows the anodic potentiodynamic polarization curves of the bodies referred to in the context of Fig. 1 in both a chlorine-containing and an acidiccontaining environment;
- Fig. 3: shows the surface defects of a body containing a coating according to the present invention (reflected by the proportion of uncoated areas in relation to the total area), a first pair of samples comprising a core body having an arithmetical mean roughness Sa of about 0.8 um and a second pair of samples comprising a core body having a Sa of lower than 0.2 um;
- Fig. 4: shows the anodic potentiodynamic polarization curves of the samples referred to in the context of Fig. 3 as well as of an uncoated body in a chlorinecontaining and acidic-containing environment; and
- Fig. 5: shows for three bodies according to the present invention the friction coefficient determined in relation to the load applied to the respective body in comparison to comparative samples containing a core body of a crystalline alloy (Ti-6Al-4V).

### Analytical details

### PVD & PECVD

The coating of the samples was carried out by a method as outlined above comprising the following steps:
1) Cleaning of the sample surface by ion bombardment using an inert gas, for example, nitrogen (N).
2) Plasma Vapor Deposition (PVD) step to deposit by ion bombardment the Cr interlayer. A bias current is applied on the target (like a polarization anode-cathode).
3) An alternative to Plasma Vapor Deposition (PVD) is the Plasma-Enhanced Chemical Vapor Deposition (PECVD) which consists of the plasma activation of precursor gases promoting the precipitation of dense thin film on the sample, for example, a silicon-based one. Then, a carbon-based gas (e.g. C₂H₂) is injected in the vacuum chamber to deposit the carbon-based thin film.

The whole process is conducted at a temperature below 250°C.

### Scanning electron microscope (SEM)

The surface of the sample bodies was observed using a SEM TESCAN VEGA 3 at an acceleration voltage of 10 keV and a working distance of 10 mm in secondary electron (SE) mode. In backscattered electron (BSE) mode the acceleration voltage was increased to 15 keV.

### Energy dispersive X-ray spectroscopy (EDXS)

The chemical compositions of the samples and the DLC coating were assessed by EDXS using an Oxford Instruments X-act Penta FET detector equipped on a Scanning Electron Microscope (SEM, TESCAN VEGA 3, TESCAN) at an acceleration voltage of 15 or 20 keV and at a working distance of 15 mm. The data was acquired and analyzed using the software Aztec One (Oxford Instruments).

### Measurement of surface defects area

Surface defects (which in this context refer to surface areas of the body sample that are not covered by the DLC coating) appear in a light colour (near white) on the SEM images in backscattered electron (BSE) mode, in contrast to the covered areas which appear in dark colour (near black)The per mille (‰) of uncovered area in relation to the total usable area of the body sample was estimated by post-treating SEM images in BSE mode on the free-access image processing program ImageJ.

### Sa (arithmetical mean height)

The surface roughness parameter Sa, defined as the arithmetical mean height to a surface, was measured using a 3D non-contact optical profiler Sensofar Neox machine (SENSOFAR). The measurement was completed on an acquisition area of 870 x 650 µm² with an objective x20 using Vertical Scanning Interferometry (VSI) technique. The acquired data was post-treated on the SensoMAP software (SENSOFAR) to extract the Sa parameter.

### Measurement of corrosion resistance

The corrosion performances of the sample bodies were investigated by electrochemical measurements in a three-electrode glass cell using a Gamry instrument 600+ potentiostat (GAMRY Instruments) with a graphite rod as counter electrode and a saturated calomel electrode (SCE) as reference electrode (potential E_{SCE} = 268 mV vs Standard Hydrogen Electrode potential at 25 °C). The working electrode consisted of a sample body connected to a copper wire on its uncoated circular face. Only the coated circular face of the sample body was in contact with the electrolyte solution, the other parts of the sample body being insulated by waterproof sheath.

The electrochemical tests were performed in two different electrolyte solutions maintained at 37°C by a water circulating temperature control unit: 1) in an aerated and neutral saline solution (pH = 7.4) composed of 0.9 weight/volume percentage concentration (w/v%) of sodium chloride (NaCl) as recommended in the ISO 10271 standard and 2) in an aerated acidic saline solution (pH = 2.3) composed of 0.9 w/v% NaCl + 1 w/v% lactic acid.

The electrochemical tests consisted of open circuit potential (OCP) measurements during 6H to reach a steady state and were followed by linear polarization measurements. The polarization curves parameters were set to a scan rate of 0.17 mV/s (millivolt per seconds), from -0.2 V vs SCE to +1 V vs SCE. To compare the corrosion performances between the samples, the electrochemical metrics E_{corr} (corrosion potential), Eₚᵢₜ (pitting potential) and I_{corr} (corrosion current density) were estimated from the anodic polarization curves. In particular, E_{corr} and I_{corr} were estimated by Tafel's extrapolation method.

### Measurement of scratch resistance

The adhesion of the DLC coating on the sample bodies was estimated through scratch tests. Scratches were achieved using a Bruker UMT Tribolab equipped with a Rockwell diamond indenter (curvature radius 200 µm) in a ramp loading mode starting from 0.5 to 15 Newton for a total scratch length of 1.5 mm. Scratch tests were performed 3 times per body sample at room temperature.

### Preparation of samples

Ingots with a targeted composition of Ti₄₀Zr₁₀Cu₃₆Pd₁₄ (in atomic%), or Ti₂₉Zr_{13.8}Cu_{34.6}Pd_{22.6} (in mass %), were prepared by arc melting highly pure elements (purities above 99.9%) in an argon atmosphere. Rods of 3.4 mm in diameter and 40 mm in length were rapidly solidified into a cylindrical water-cooled copper mold by injection casting. The composition of all the rods was controlled by Energy-Dispersive X-ray spectroscopy (EDX) on a Scanning Electron Microscope (TESCAN VEGA 3).

From the rods obtained, smaller cylinders of 3.4 mm in diameter and 3 mm in length were cut. In the middle of each cylinder, a groove was machined on its entire circumference so that the cylinders can later be held in place by a wire during the DLC process. The groove was 0.5 µm deep with a diameter of 1 µm.

For a first set of cylinder samples, core body (CBI_{A}) was grounded and polished using silicon carbide (SiC) papers with grains of #1200 and #2500 grits to obtain a surface having an average mean roughness Sa of less than 0.2 µm. Surface polishing was conducted on a MetaServ 250 Grinder-Polisher (BUEHLER) at a rotation speed of 150 rounds per min (rpm) for 5 minutes for each SiC paper grit on both circular faces of CBI₁ and CBI₂. For a second set of samples, core body (CBI_{B}) having an average mean roughness Sa of about 0.8 µm was prepared.

Prior to the DLC deposition, all the core bodies CBI_{A} and CBI_{B} were cleaned successively in an acetone bath followed by an ethanol bath (5 min per ultrasonic bath). Then, all of the core bodies were subjected to PECVD treatment, by which in a first step an intermediate layer containing chromium as a main component was deposited on the surface of the core body by PVD. On the intermediate layer, an inner silicon-based DLC layer region, which contains silicon as a main component, was applied by PECVD, followed by the application of an outer DLC layer onto the inner DLC layer deposited by PECVD, said outer DLC layer being a carbon-based DLC layer which contains carbon as a main component (regarding the details of the method, see description under "PVC & PECVD" above). Lastly, sample bodies CBI_{A} and CBI_{B} were cut in half at their groove using a micro-cutting machine and without damaging the circular faces coated by DLC. Cutting CBI_{A} and CBI_{B} resulted in 4 disks having a diameter of 3.4 mm and a length of 1 mm.

Thus, sample bodies B_{invA1} and B_{invA2} (based on "smooth" core body CBI_{A}) and sample bodies B_{invB1} and B_{invB2} (based on "rough" core bodies CBI_{B}) according to the present invention were achieved.

For reasons of comparison, uncoated sample B_{compA} was prepared in a similar way than B_{invA1} and B_{invA2} and another uncoated sample B_{compB} was prepared in a similar way than B_{invB1} and B_{invB2}. Specifically, B_{compA} and B_{compB} differ from B_{invA1} and B_{invA2} and from B_{invB1} and B_{invB2}, respectively, in that the DLC coating step was not applied.

An additional comparative sample B_{compC} made of Ti-6Al-4V was prepared in a similar way than B_{invA1} and B_{invA2.}

A list of the samples referred to above, in which the material used, the surface finish and the (non-) presence of the DLC coating is summarized, is given in Table 1 below, in which also the reference to the respective figure is indicated.

**Table 1**

| **Samples ID** | **Material** | **Surface finish** | **DLC (Y/N)** | **Fig.** |
|---|---|---|---|---|
| B_{compB} | BMG | As-machined | No | 1 |
| B_{invB1} | | | Yes | |
| B_{compA} | Ti₄₀Zr₁₀Cu₃₆Pd₁₄ | Polished | No | 2 |
| B_{invA1} | | | Yes | |
| B_{invA1} | | Polished | Yes | 3 |
| B_{invB2} | | As-machined | | |
| B_{compA} | | Polished | No | 4 |
| B_{invA1} | | Polished | Yes | |
| B_{invB2} | | As-machined | | |
| B_{invA2} | | Polished | Yes | 5 |
| B_{compC} | Ti-6Al-4V ELI | | | |

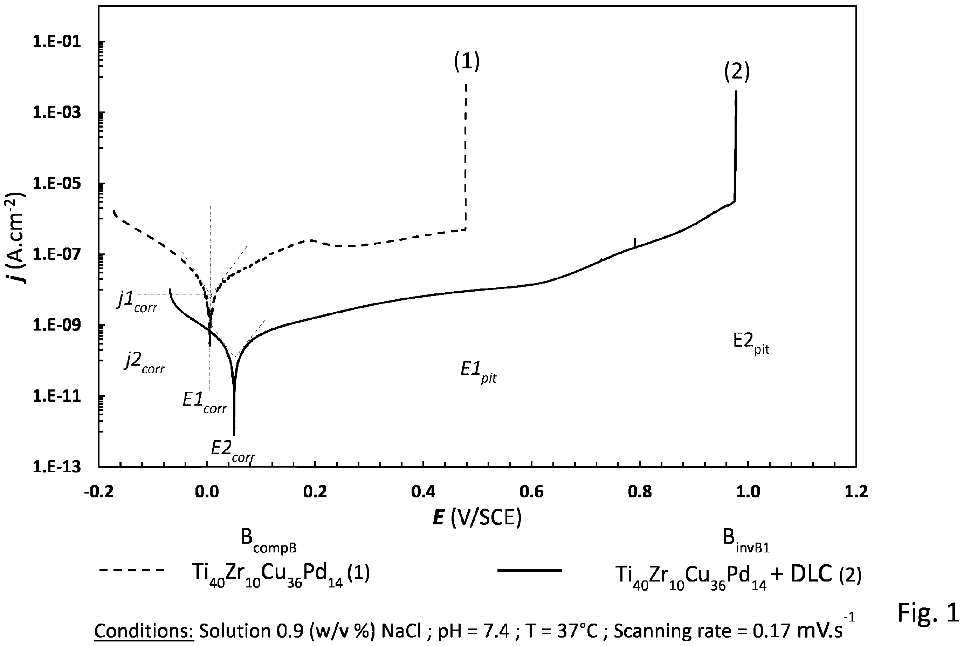

As shown in Fig. 1 (relating to the corrosion resistance measurement at physiological pH), the sample of the present invention B_{invB1} showed a corrosion current density (I_{corr}) far lower than the one determined for the uncoated comparative sample B_{compB}.

In addition, an ΔE (corresponding to Eₚᵢₜ - E_{corr}) was determined for the sample of the present invention, which is far higher (by a factor of about 2) than the one determined for the uncoated comparative sample. Both these results are indicative of a higher corrosion resistance and a reduced susceptibility to pitting corrosion of the sample according to the present invention over the comparative sample.

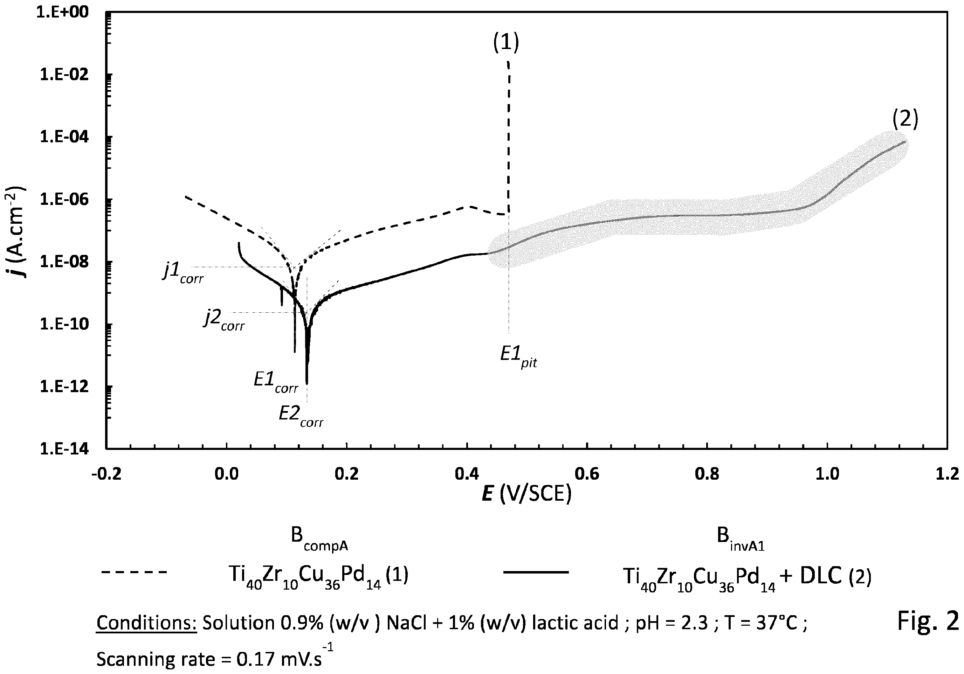

A similar result in terms of a higher corrosion resistance and a reduced pitting corrosion susceptibility of the sample of the present invention B_{invA1} is shown in Fig. 2 (relating to the corrosion resistance measurement in an acidic environment). According to Fig. 2, no pitting corrosion for the sample of the present invention B_{invA1} was determined at all, showing that the coating according to the present invention is also protective in an environment which is both chlorine-containing and acidic-containing.

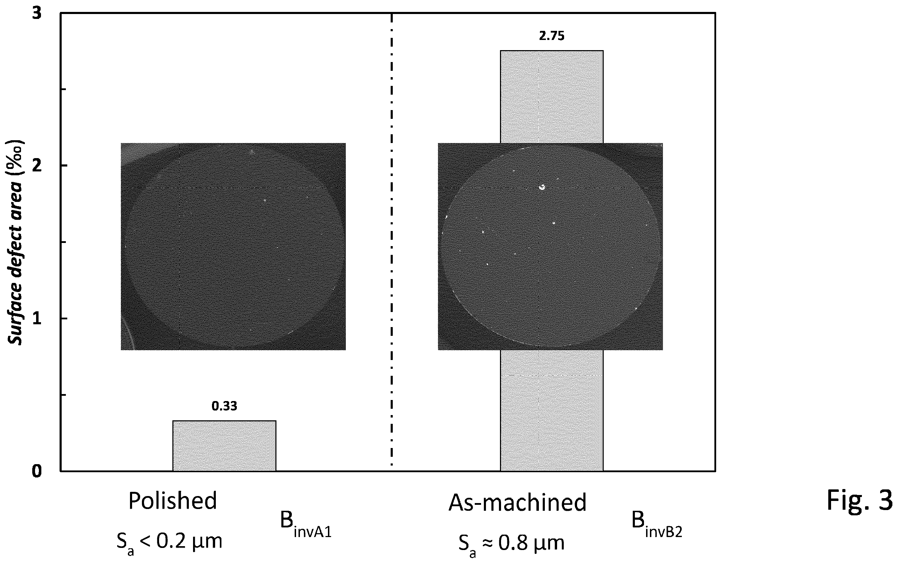

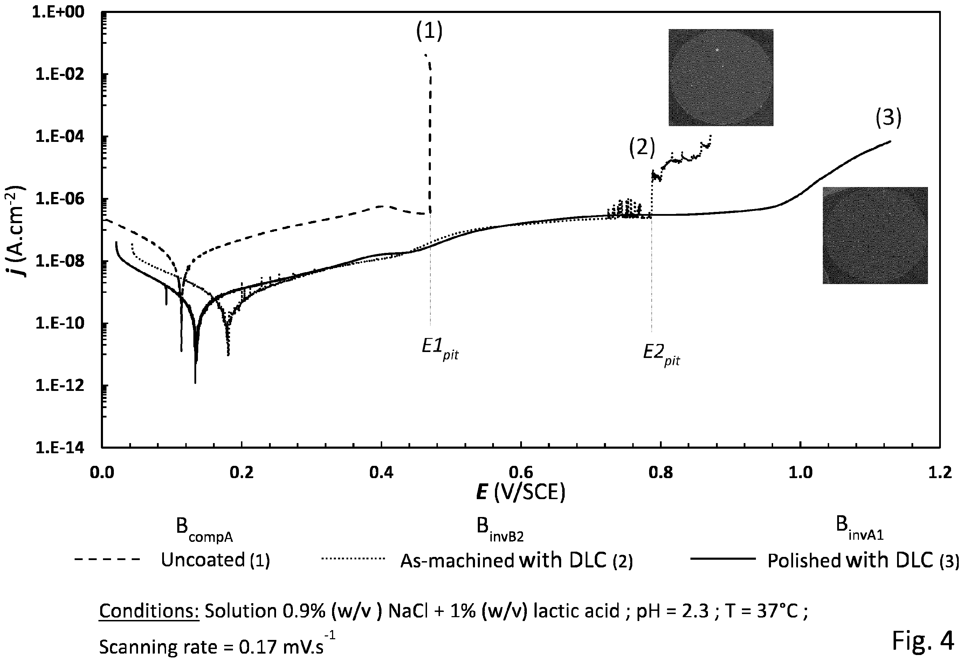

As discussed above, it was found in the context of the present invention that the lowering of the material's susceptibility to pitting corrosion is to a certain degree also governed by the surface topography of the core body. This is illustrated by Fig. 4, showing for sample B_{invA1} (based on core body CBI_{A} having a relatively smooth surface) a lower sensitivity to pitting corrosion as compared to samples B_{invB2} (based on core body CBI_{B} having a relatively rough surface). The surface defects of samples B_{invA1} and B_{invB2} are given in Fig. 3, showing for sample B_{invB2} substantially higher surface defects than for B_{invA1}.

Although for sample B_{invB2} (exhibiting a relative high degree of surface defects) a slightly higher I_{corr} and a higher ΔE were determined than for sample B_{invA1}, also sample B_{invB2} showed a higher corrosion resistance and a further reduced pitting corrosion susceptibility over the (uncoated) comparative sample B_{compA}, as shown in Fig. 4.

### Measurement of scratch resistance

For determining the scratch resistance, sample body B_{invA2} according to the present invention and of comparative sample B_{compC} were subjected to a scratch resistance test using a Rockwell indent and applying a ramp load from 0.5 to 15 N for a scratch length of 1.5 mm.

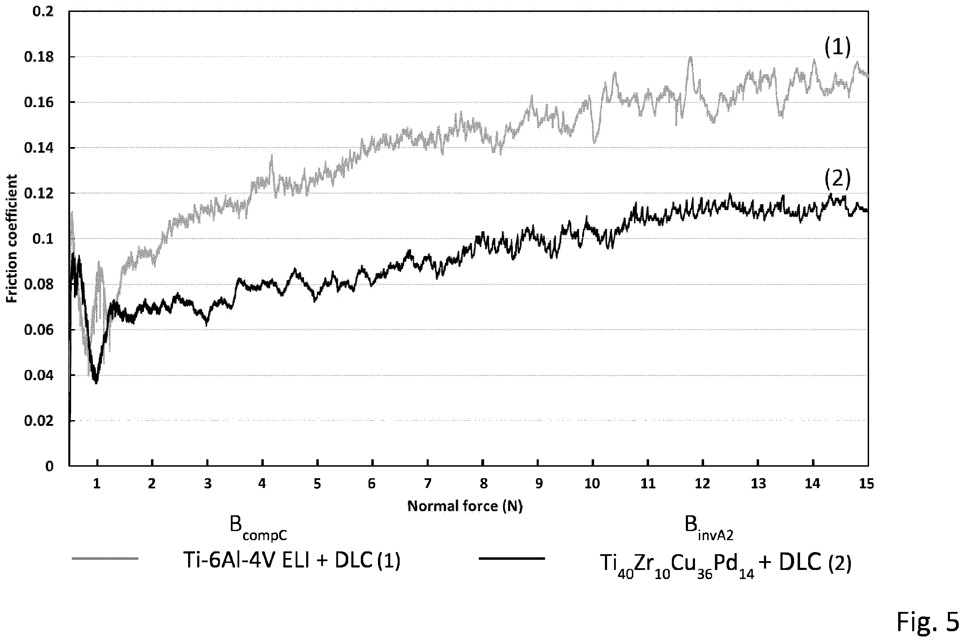

The results are presented in Fig. 5, showing for the body B_{invA2}, according to the present invention, a friction coefficient lower than the one of the comparative sample B_{compC} containing a core body of a crystalline alloy (Ti-6Al-4V).

These results give thus clear evidence that an improved adhesion of the DLC layer coating is achieved when applied on a core body of an amorphous alloy (according to the present invention) than when applied on a crystalline alloy. Hence, a medical device or medical device component can be achieved, which even when subjected to high forces exhibits a high scratch resistance and, hence, a low susceptibility of the coating to be scratched or to delaminate.

## Claims

1. A medical device or a medical device component comprising a core body made of an amorphous alloy, wherein the main alloy component of the amorphous alloy is selected from the group consisting of titanium copper (TiCu) and zirconium copper (ZrCu), **characterized in that** the device further comprises a coating formed on at least a part of a surface of the core body, the coating containing a diamond-like carbon (DLC) layer.

2. The medical device or medical device component according to claim 1, wherein the amorphous alloy is a Bulk Metallic Glass.

3. The medical device or medical device component according to any of the preceding claims, wherein the amorphous alloy contains titanium and copper as the main alloy components and further contains at least one secondary alloy component selected from the group consisting of zirconium (Zr), palladium (Pd), and mixtures thereof.

4. The medical device or medical device component according to claim 3, wherein the amorphous alloy further contains at least one tertiary alloy component selected from the group consisting of iron (Fe), gallium (Ga), tin (Sn), silicon (Si), yttrium (Y), silver (Ag), scandium (Sc), sulfur (S), niobium (Nb), hafnium (Hf), zinc (Zn), tantalum (Ta), and mixtures thereof.

5. The medical device or medical device component according to claim 4, wherein the amorphous alloy contains 15-55 weight-% of titanium, 1-45 weight-% of copper, 0-30 weight-% of zirconium, and 0-30 weight-% of palladium, 0-10 weight-% of silicon, 0-20 weight-% of iron, 0-10 weight-% of zinc, and 0-20 weight-% of silver, based on the total weight of the amorphous alloy.

6. The medical device or medical device component according to claim 5, wherein the amorphous alloy contains
20-45 weight-%, preferably 25-35 weight-%, of titanium,
15-45 weight-%, preferably 30-40 weight-%, of copper,
5-30 weight-%, preferably 10-20 weight-% of zirconium,
0-30 weight-%, preferably 0-25 weight-% of palladium,
0-15 weight-%, preferably 0-10 weight-% of tin,
0-2 weight-%, preferably 0-1 weight-% of silicon,
0-10 weight-%, preferably 0-5 weight-% of iron,
and 0-15 weight-%, preferably 0-10 weight-% of silver, based on the total weight of the amorphous alloy.

7. The medical device or medical device component according to any of claims 3 to 6, wherein the amorphous alloy is of the composition Ti₄₀Zr₁₀Cu₃₆Pd₁₄, the suffixes relating to the atomic percentages of the respective components.

8. The medical device or medical device component according to any of the preceding claims, wherein the coating is formed directly on the surface of the core body.

9. The medical device or medical device component according to any of the preceding claims, wherein the coating further contains an intermediate layer formed between the DLC layer and the surface of the core body, the intermediate layer comprising chromium.

10. The medical device or medical device component according to any of the preceding claims, wherein the DLC layer contains a silicon-based inner DLC layer region and a carbon-based outer DLC layer region.

11. The medical device or medical device component according to claim 10, wherein the proportion of silicon contained in the inner DLC layer region decreases gradually in the direction of the outer DLC layer region and the proportion of carbon contained in the outer DLC layer region decreases gradually in the direction of the inner DLC layer region.

12. The medical device or medical device component according to any of the preceding claims, wherein the surface of the core body on which the DLC coating is formed has an arithmetical mean roughness Sa of less than 1 um, preferably less than 0.5 um, more preferably less than 0.3 um, and even more preferably less than 0.2 µm.

13. The medical device or medical device component according to any of the preceding claims, wherein the medical device is a surgical device, a dental implant, a prosthesis, an abutment, a dental implant supported prosthesis or abutment, a screw for use in a dental implant supported prosthesis or abutment, a surgical instrument, a dental instrument, a dental driving tool, a handpiece for use in surgical or dental procedures, a motor for use in surgical procedures, or a motor for use in dental procedures.

14. A process for preparing a medical device or medical device component according to any of the preceding claims, wherein the process comprises the steps of
a. providing a core body made of an amorphous alloy, wherein the main alloy component of the amorphous alloy is selected from the group consisting of titanium copper (TiCu) and zirconium copper (ZrCu), and
b. applying onto the surface of the core body a coating containing a diamond-like carbon (DLC) layer.

15. The process according to claim 14, wherein the diamond-like carbon (DLC) layer is applied onto the surface of the core body using a vapor deposition method at a temperature of below 300°C, preferably of below 250°C.
